(19) Europäisches Patentamt / European Patent Office / Office européen des brevets

(11) EP 2 573 099 A1

(12) **EUROPEAN PATENT APPLICATION**
published in accordance with Art. 153(4) EPC

(43) Date of publication:
**27.03.2013 Bulletin 2013/13**

(21) Application number: **11783453.1**

(22) Date of filing: **13.05.2011**

(51) Int Cl.:
***C07H 15/04*** *(2006.01)* ***C07H 1/00*** *(2006.01)*

(86) International application number:
**PCT/JP2011/061049**

(87) International publication number:
**WO 2011/145519 (24.11.2011 Gazette 2011/47)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.05.2010 JP 2010113066**

(71) Applicants:
- **Nagasaki University**
  **Nagasaki-Shi, Nagasaki 852-8521 (JP)**
- **JX Nippon Oil & Energy Corporation**
  **Chiyoda-ku**
  **Tokyo 100-8162 (JP)**

(72) Inventors:
- **MOTOKUCHO, Suguru**
  **Nagasaki-shi**
  **Nagasaki 852-8521 (JP)**
- **INOUE, Toshio**
  **Tokyo 100-8162 (JP)**

(74) Representative: **Elend, Almut Susanne et al**
**Venner Shipley LLP**
**Byron House**
**Cambridge Business Park**
**Cowley Road**
**Cambridge CB4 0WZ (GB)**

(54) **METHODS FOR PRODUCING GLUCOSIDE**

(57) The present invention relates to methods for producing glucosides directly from glucose or a polysaccharide comprising glucose as a structural unit. The present invention provides a method comprising reacting glucose or a polysaccharide comprising glucose as a structural unit with a compound represented by R-OH in the presence of a supercritical or subcritical carbon dioxide to produce glucosides and a method comprising dissolving or suspending glucose or a polysaccharide comprising glucose as a structural unit in an organic solvent containing a compound represent by R-OH and reacting the glucose or polysaccharide with the compound represented by R-OOH in the presence of a supercritical or subcritical carbon dioxide to produce glucosides.

[Fig. 1]

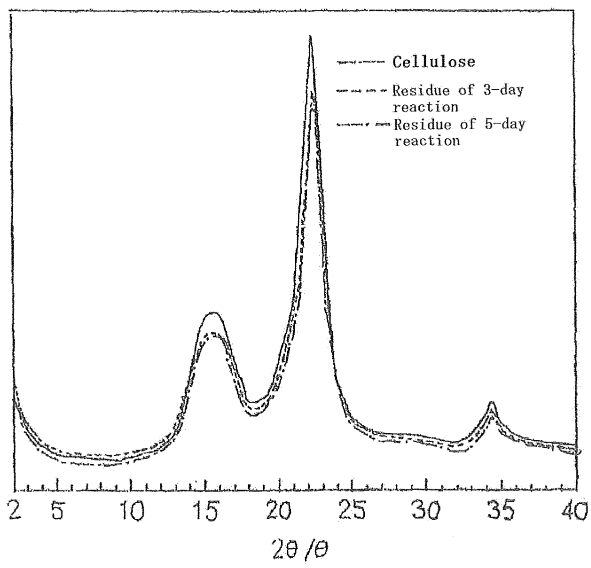


EP 2 573 099 A1

## Description

### Technical Field

The present invention relates to methods for producing glucosides. More specifically, the present invention relates to a method for producing glucosides by reacting glucose or a polysaccharide including glucose as a structural unit with a hydroxyl-containing compound in the presence of a supercritical or subcritical carbon dioxide.

### Background Art

A method for producing glucosides from glucose using an acid catalyst or enzyme is known. However, there is a problem that the method using an acid catalyst requires the steps of addition and removal thereof (Patent Literatures 1 and 2) while the method using an enzyme requires a post treatment thereof (Patent Literatures 3 and 4).

Cellulose decomposition technology is one of the known techniques using a supercritical fluid, and examples of such techniques include:

(a) decomposition of cellulose with a super(sub)critical fluid (Patent Literature 5);
(b) decomposition of cellulose with a supercritical methanol (Non-Patent Literature 1);
(c) decomposition of cellulose with a supercritical carbon dioxide and water (Patent Literature 6, Non-Patent Literature 2); and
(d) decomposition of cellulosic biomass with an aqueous solution of a super(sub)critical aliphatic alcohol (Patent Literature 7).

The above (a) discloses a method wherein cellulose is decomposed through glucose and 5-hydroxymethylfurfural to various carboxylic acids. The above (b) through (d) also each discloses a method for producing glucose by decomposing cellulose, which cannot avoid the formation of oligosaccharide by decomposition of cellulose, the formation of products such as levoglucosan, 5-hydroxymethylfurfural, furfural and levulinic acid by isomerizing glucose and the formation of compounds through thermal decomposition of cellulose.

Furthermore, a technology of decomposing a polymer material through methanolysis with a supercritical carbon dioxide and methanol to monomers is known to be applied to a polyurea with a specific structure (Non-Patent Literature 3), but there is no disclosure about methanolysis of a polysaccharide possibly involving oligosaccharide formation or isomerization.

### Citation List

### Patent Literatures

Patent Literature 1: Japanese Patent Laid-Open Publication No. 2-275892
Patent Literature 2: Japanese Patent Laid-Open Publication No. 9-31089
Patent Literature 3: Japanese Patent Laid-Open Publication No. 2002-17395
Patent Literature 4: Japanese Patent Laid-Open Publication No. 2002-17396
Patent Literature 5: Japanese Patent Laid-Open Publication No. 5-31000
Patent Literature 6: Japanese Patent Laid-Open Publication No. 2006-263527
Patent Literature 7: Japanese Patent Laid-Open Publication No. 2005-296906

### Non-Patent Literatures

Non-Patent Literature 1: "Cellulose", 8, 189 (2001) by Y. Ishikawa and S. Saka
Non-Patent Literature 2: "Polymer Preprints, Japan", 58 (2), 5387 (2009), by Hiroshi Ichiyanagi, Mutsuhisa Furukawa, Ken Kojio, Suguru Motokucho
Non-Patent Literature 3: "Polymer Preprints, Japan", 56 (1), 2359 (2007) by Suguru Motokucho, Shingo Mukai, Ken Kojio, Mutsuhisa Furukawa

## Summary of Invention

### Technical Problem

The present invention provides a method for producing glucosides from glucose or a polysaccharide including glucose as a structural unit, which does not require addition or removal of an acid catalyst or an enzyme.

### Solution to Problems

As the result of extensive study and research conducted by the inventors, the present invention has been accomplished on the basis of the finding that reaction of glucose or a polysaccharide including glucose as a structural unit with a compound containing a hydroxyl group represented by R-OH in the presence of a supercritical or subcritical carbon dioxide results in the production of glucosides in a high selectivity and a high purity.
That is, the present invention is as follows:

[1] a method for producing glucosides comprising reacting glucose or a polysaccharide comprising glucose as a structural unit with a hydroxyl-containing compound represented by formula (1) below in the presence of a supercritical or subcritical carbon dioxide to produce glucosides represented by formula (2) below:

R-OH (1)

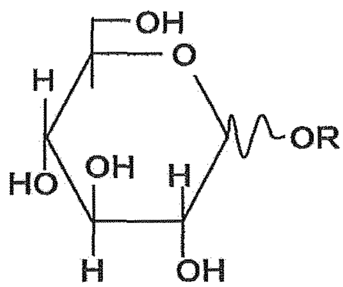

(2)

(in formula (1), R is any substituent, provided that the hydroxyl group in formula (1) bonds to a carbon atom of R and R is excluded from the substituents making the compound of formula (1) sugar, and in formula (2), the wavy line indicates an α-configuration or a β-configuration, and R is as defined with respect to R in formula (1));

[2] a method for producing glucosides comprising dissolving or suspending glucose or a polysaccharide comprising glucose as a structural unit in an organic solvent containing a hydroxyl-containing compound represent by formula (1) below and reacting the glucose or polysaccharide with the hydroxyl-containing compound represented by formula (1) in the presence of a supercritical or subcritical carbon dioxide to produce glucosides represented by formula (2) below:

R-OH (1)

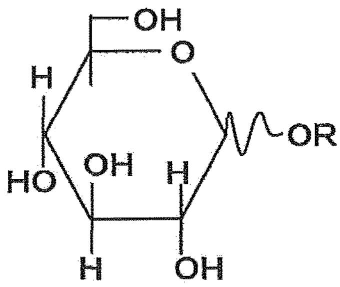

(2)

(in formula (1), R is any substituent, provided that the hydroxyl group in formula (1) bonds to a carbon atom of R and R is excluded from the substituents making the compound of formula (1) sugar, and in formula (2), the wavy line indicates an α-configuration or a β-configuration, and R is as defined with respect to R in formula (1));

[3] a method according to [1] or [2] above wherein the polysaccharide comprising glucose as a structural unit comprises any one selected from amylose, amylopectin and cellulose;
[4] a method according to any one of [1] to [3] above wherein the polysaccharide comprising glucose as a structural unit comprises starch;
[5] a method according to any one of [1] to [4] above wherein the hydroxyl-containing compound represented by formula (1) is an alkylalcohol; and
[6] a method according to any one of [1] to [5] above wherein the hydroxyl-containing compound represented by formula (1) is methanol.

**Advantageous Effects of Invention**

The method of the present invention can produce glucosides from glucose or a polysaccharide including glucose as a structural unit without adding and removing an acid catalyst or an enzyme. Furthermore, since a polysaccharide including glucose as a structural unit as it is can be used as a raw material, the method of the present invention can produce glucosides directly therefrom without using glucose, which is expensive.

**Brief Description of the Drawings**

Fig. 1 shows the X-ray diffraction patterns of cellulose and residues after being reacted for 3 days and 5 days.
Fig. 2 shows the $^{13}$C-NMR spectrums of α-methylcellulose and a methanol soluble matter after being reacted for 5 days.
Fig. 3 shows the enlarged $^{13}$C-NMR spectrums in the vicinities of δ=100ppm of α-methylcellulose and a methanol soluble matter after being reacted for 5 days.
Fig. 4 shows the HPLC analysis result of an oligosaccharide standard solution.
Fig. 5 shows the HPLC analysis result of a methanol soluble matter after being reacted for 3 days.
Fig. 6 shows the HPLC analysis result of a methanol soluble matter after being reacted for 3 days.
Fig. 7 shows the HPLC analysis result of an ethanol soluble matter produced from starch.

**Description of Embodiments**

The present invention will be described in more detail below.
Glucose used in the present invention may be α-glucose, β-glucose or a mixture thereof.
The term "polysaccharide comprising glucose as a structural unit" used herein refers to a group of compounds where sugars comprising glucose connects to each other via glycoside bonds.
No particular limitation is imposed on the polysaccharide comprising glucose as a structural unit if it comprises glucose as a structural unit. The polysaccharide may be any of those occurring in nature or those produced by synthesis. Furthermore, no particular limitation is imposed on its polymerization degree or bonding form such as 1,4-bond, 1,6-bond, α-bond and β-bond. The polysaccharide may be cyclodextrin.
Among them, preferred are cellulose and amylose, which have a linear chain structure based on 1,4 bonds and amylopectin, which consists of mainly 1,4 bonds and branchings taking place with 1,6 bonds because they contain a large amount of glucose, are easily available due to their existence in large amount in nature, and easily decomposable due to their simple structures. Since intermolecular interaction is preferably small to be easily decomposable, particularly preferred are amylose and amylopectin due to their low crystallinity.
These may be used alone or in combination. Alternatively, biomass containing cellulose, chemically treated products (for example pulp) or milled products thereof may be used as it is. Further alternatively, starch containing amylose or amylopectin may be used as it is. The format is preferably powder, which has a large surface area to effect decomposition efficiently.

The glucosides produced by the present invention are o-glucosides that are compounds derived by substituting the hemiacetal hydroxyl group (also referred to as "glucoside hydroxyl group") of sugar with a substituent derived by removing hydrogen from aglycon, which is a non-sugar component, among which compounds the atom bonding to an anomeric carbon is oxygen.

According to a first aspect of the present invention, glucose or a polysaccharide comprising glucose as a structural unit is reacted with R-OH of formula (1) in the presence of a supercritical or subcritical carbon dioxide to produce glucosides represented by formula (2) above.

"Supercritical carbon dioxide" refers to carbon dioxide at a pressure of 7.4 MPa or greater and a temperature of 31°C or higher while "subcritical carbon dioxide" refers to carbon dioxide not meeting these requirements but around the pressure and temperature.
The inventors of the present invention assume that a supercritical or subcritical carbon dioxide has the following functions. At first, it is assumed that a supercritical or subcritical carbon dioxide penetrates through a polysaccharide and weakens the intermolecular interaction therein and thus that due to this effect, the field for the glucoside-formation reaction concerning the method of the present invention is ensured. The second is a function that a supercritical or subcritical carbon dioxide interacts with the compound represented by R-OH in formula (1) to form "$H^+$" and "$R\text{-}O^-$" as shown in formula (3) below. In formula (3), $scCO_2$ indicates a supercritical or subcritical carbon dioxide.

$$scCO_2 + R\text{-}OH \leftrightarrow scCO_2 \cdot\cdot R\text{-}O^- + H^+ \qquad (3)$$

Carbon dioxide is known to be a compound, that is poor in reactivity, but a supercritical or subcritical carbon dioxide is empirically known to have reactivity or interactivity with other compounds. The inventors assume that "$H^+$" formed in formula (3) initiates and proceeds with the decomposition of a polysaccharide including glucose as a structural unit and that the polysaccharide decomposes to an oligosaccharide and then a monosaccharide while "$R\text{-}O^-$" is incorporated in the form of aglicone into glucose or the polysaccharide including glucose to produce glucosides represented by formula (2). It is also assumed that in the presence of a solvent, the polysaccharide is dissolved in the solvent at the stage of being decomposed to an oligosaccharide and then decomposed to a monosaccharide in the solvent to produce glucosides represented by formula (2).

The reaction is preferably carried out at a temperature or below at which a polysaccharide is thermally decomposed. This condition can suppress a polysaccharide from decomposing causing the formation of an oligosaccharide and can produce glucosides at a high selectivity.

The reaction is preferably carried out at a temperature lower than and/or pressure lower than the supercritical conditions for R-OH of formula (1) and particularly preferably at both a temperature and pressure which are lower than the supercritical conditions. The reaction under these conditions does not form a various ions such as "$H^+$" in large amounts derived from R-OH itself or intermolecular interaction thereof in the supercritical or subcritical state and thus proceeds under mild conditions where generation of "$H^+$" resulting from formula (3) mainly occurs thereby suppressing both decomposition of a polysaccharide causing the formation of an oligosaccharide and isomerization of the resulting oligosaccharide, glucose and glucoside. As the result, glucosides can be produced at a high selectivity.

No particular limitation is imposed on the reaction time, which may be at least sufficient to produce glucosides represented by formula (2) according to the present invention. For example, the time is usually 1 hour or longer, preferably 10 hours or longer, more preferably 20 hours or longer, more preferably 30 hours or longer. For the upper limit, the reaction may be carried out until glucose or a polysaccharide including glucose as a structural unit, i.e., the raw material is completely decomposed. In general, the upper limit is preferably 10 days or shorter in view of economy.
The ratio of R-OH to be used is preferably excess with respect to the glucose or the glucose in the polysaccharide and is at least stoichiometry, preferably 5 molar equivalents or more, more preferably 10 molar equivalents or more.

According to a second aspect of the present invention, glucose or a polysaccharide comprising glucose as a structural unit is dissolved or suspended in an organic solvent containing R-OH of formula (1) in the presence of a supercritical or subcritical carbon dioxide and then reacted with R-OH of formula (1) in the presence of the supercritical or subcritical carbon dioxide to produce glucosides represented by formula (2).
That is, the glucose and glucosides formed by the present invention has a possibility of being isomerized due to "$H^+$"

formed as shown in formula (3), but the isomerization can be suppressed by solvation thereof with the organic solvent, i.e., giving cage effect so as to stabilize the glucose and glucosides. As the result, glucosides can be produced at a high selectivity.

No particular limitation is imposed on the organic solvent solvating glucose or glucosides. Particularly preferably in view of the reaction efficiency, a solvent containing R-OH in an excess molar amount in respect of the glucose in a polysaccharide is used as a solvent or suspension medium of the polysaccharide or a solvent of glucosides so that the solvent solvates glucose or glucosides.

If "$H^+$" generated from the excess R-OH causes isomerization, the R-OH is preferably diluted with another solvent, particularly a non-protonic organic solvent to effect stabilization by solvating glucose or glucosides with these plurality of solvents.

When the R-OH is solid, it is preferably dissolved or suspended in an organic solvent, particularly a polar non-protonic organic solvent to effect stabilization by solvating glucose or glucosides with the dilution solvent.

Examples of the polar non-protonic organic solvent include ethylene glycol dimethyl ether, ethylene glycol methyl lethyl ether, diethylene glycol dimethyl ether, diethylene glycol methyl ethyl ether, triethylene glycol dimethyl ether, tetraethylene glycol dimethyl ether, ethylene glycol diethyl ether, diethylene glycol diethyl ether, 1,3-dimethoxypropane, 1,2-dimethoxypropane, propylene glycol dimethyl ether, dipropylene glycol dimethyl ether, dioxane, dimethyl carbonate, ethyl methyl carbonate, diethyl carbonate, ethylene carbonate, propylene carbonate, 2,3-dimethyethylene carbonate, butylne carbonate, acetonitrile, methoxy acetonitrile, propionitrile, butyrolactone, valerolactone, dimethoxyethane, sulforane, methylsulforane, sulfolene, dimethyl sulfone, ethylmethyl sulfone, and isopropyl methyl sulfone. A mixture of any two or more of these compounds may be used.

The conditions for the reaction of glucose or a polysaccharide comprising glucose as a structural unit with R-OH dissolved or suspended in an organic solvent in the presence of a supercritical or subcritical carbon dioxide are the same as those described with respect to the above first aspect of the present invention.

The reactions in the present invention are preferably carried out under conditions where a supercritical or subcritical carbon dioxide is sealed but may be carried out, circulating a supercritical or subcritical carbon dioxide.

In R-OH that is formula (1) used in the present invention, R is any substituent. No particular limitation is imposed on the substituent if in formula (1), the hydroxyl group bonds to a carbon atom of R and R-OH itself is not sugar. However, compounds of the formula R-OH are preferably compounds that are small in steric hindrance or compounds that are large in dissociation constant pKa.

Examples of R in formula (1) include alkyl, aralkyl, aryl, and alkylaryl groups, having 1 to 30, preferably 1 to 20, more preferably 1 to 12 carbon atoms.

Examples of compounds represented by R-OH, i.e., formula (1) include aliphatic alcohols, benzyl alcoholic compounds as well as phenols wherein the hydroxyl group bonds to an aromatic hydrocarbon atom.

Examples of aliphatic alcohols include methanol, ethanol, ethylene glycol, diethylene glycol, triethylene glycol, tetraethylene glycol, pentaethylene glycol, propanol, isopropanol, allyl alcohol, propargyl alcohol, propylene glycol, trimethylene glycol, n-butyl alcohol, sec-butyl alcohol, ter-butyl alcohol, crotyl alcohol, methallyl alcohol, pentyl alcohol, dimethylallyl alcohol, isopentenyl alcohol, neopentyl glycol, trimethylolethane, pentaerythritol, dipentaerythritol, tripentaerythritol, hexanol, pinacolyl alcohol, pinacol, hexylene glycol, trimethylolpropane, heptanol and alcohols having 7 to 20 carbon atoms. Among these alcohols, methanol is particularly preferable.

Examples of benzyl alcoholic compounds include benzyl alcohol, salicyl alcohol, anisyl alcohol, anisic alcohol, gentisyl alcohol, protocatechuyl alcohol, vanillyl alcohol, veratryl alcohol, cuminyl alcohol, phenethyl alcohol, homovanillyl alcohol, homoveratryl alcohol, hydrocinnamyl alcohol, $\alpha$-cumyl alcohol, cinnamyl alcohol, coniferyl alcohol, sinapyl alcohol, benzhydryl alcohol, trityl alcohol, hydrobenzoin, benzopinacol, phthalyl alcohol, isophthalyl alcohol, and terephthalyl alcohol.

Examples of compounds of formula (1) wherein the hydroxyl group bonds to an aromatic hydrocarbon atom include phenol, cresol, xylenol, florol, pseudocumenol, mesitol, prehnitenol, isodurenol, durenol, chavicol, anol, thymol, carvacrol, pyrocatechol, resorcinol, hydroquinone, pyrogallol, phloroglucinol, orcinol, toluhydroquinone, o-xylohydroquinone, m-xylohydroquinone, p-xylohydroquinone, pseudocumohydroquinone, thymohydroquinone, durohydroquinone, olivetol, bisphenol-A, and diethylstilbestrol.

Examples other than the above-described R-OH include natural products such as monoterpene alcohols (for example, linalool), terpennoids (for example, retinol), and alcohols having a lactone structure (for example, ascorbic acid).

Glucosides produced by the method of the present invention can be used for various applications such as detergent intermediates (methylglucoside), food additives (ethylglucoside), non-ionic surfactants (n-octylglucoside, n-decylglucoside), skin-lightening agents (arbutin), pain relievers (salicin), dyes (indican), and supplements (ascorbyl glucoside) depending on the chemical structure of the substituent R.

In the present invention, a sugar wherein aglicone is introduced to the anomer carbon is not limited to glucose but may be xylose and galactose.

## Examples

The present invention will be described with reference to the following examples in more detail but is not limited thereto.

[Example 1]

(Reaction)

Into a glass container were added 5 g of cellulose ("Avicel") and 20 ml of methanol, and then the container was placed in a 200 mL stainless-steel pressure resistant reactor equipped with a pressure gauge and a rupture type relief valve (TVS-N2-200 portable reactor, manufactured by Taiatsu Techno) so that the mixture was stirred with a stirrer and allowed to suspend.
After the pressure resistant reactor was sealed, a liquefied $CO_2$ was introduced thereinto, followed by heating with a heater so that the temperature and pressure inside the reactor were 180°C and 8 MPa thereby allowing the carbon dioxide to be in the supercritical state.
This state was kept for 3 days (72 hours) and 5 days (120 hours).
The supercritical temperature and pressure of methanol are 240°C and 8 MPa.

(Residue Analysis)

After the predetermined periods of time passed, the glass container was taken out and the content therein was filtered to measure the weight of the residue and carry out a wide-angle X-ray diffraction (WAXD) measurement. The cellulose decomposition rate was calculated using the following formula.

Cellulose decomposition rate (%)

=[(weight of charged cellulose-residue weight)/(weight of charged cellulose)] x 100

As the result, the decomposition rates of the cellulose after 3 day and 5 day reactions were found to be 13.2% and 20.3 percent, respectively.

The comparison of characteristics of the residue and charged cellulose ("Avicel") were carried out by comparing their wide-angle X-ray diffraction (WAXD) patterns. The measurement was carried out using RINT-2200 X-ray diffraction device (manufactured by Rigaku Corporation) under conditions where the diffraction angle 2θ=5 to 30°, the X-ray tube voltage was 40 kV, the X-ray tube current was 40 mA, the sampling time was 4 seconds, and the step width was 0.04°.
Fig. 1 shows the X-ray diffraction patterns of the cellulose and the residue after the 3 day reaction and 5 day reaction.
As apparent from Fig. 1, two diffractions at 15.7° and 22.5° assigned to the crystal of the cellulose and the halo patterns of the amorphia overlap those of the residues, and no significant difference in the whole comparison of the patterns was found. Therefore, it is confirmed that in the present invention, a methanol soluble component was produced without giving the cellulose significant change.

(Soluble matter analysis 1: NMR spectrum analysis)

The methanol was distilled out from the 5 day reaction filtrate with a rotary evaporator and then dried under vacuum with a vacuum pump for 15 hours to give a methanol soluble matter.
Part of the methanol soluble matter was dissolved in deuterated water to carry out the carbon nuclear magnetic resonance ($^{13}C$-NMR) measurement. The measurement was carried out with a superconducting multinuclear magnetic resonator "JNM-GC400" (manufactured by JEOL Ltd.,) at 100 MHz and cumulated number of 2048 times. Fig. 2 shows $^{13}C$-NMR spectra.
The vicinity of δ=100 ppm which corresponds to the anomeric carbon was enlarged (Fig. 3).
As a comparative sample, the same measurement was carried out for a commercially available α-methyl glucoside.
From the comparison of the both shown in Fig. 2, the methanol soluble matter was found to include substantially only methyl glucoside.
From the comparison of the enlarged views of the vicinity of δ=100 ppm of the both shown in Fig. 3, the methanol soluble

matter was found to be a mixture including substantially α-methyl glucoside and β-methyl glucoside.

(Soluble matter analysis 2: HPLC analysis)

As an oligosaccharide standard, D-(+)-glucose, cellobiose, cellotriose, cellotetraose, and cellopentaose were each weighed and then dissolved in purified water to prepare oligosaccharide aqueous solutions each containing the respective component at a concentration of 10 mg/mL. Cellohexaose was weighed and dissolved in purified water to prepare an oligosaccharide aqueous solution containing cellohexanose at a concentration of 5 mg/ml. By mixing 20 μL of each of the 10 mg/mL oligosaccharide aqueous solutions, 40 μL of the 5 mg/mL oligosaccharide aqueous solution and 60 μL of acetonitrile was prepared an oligosaccharide standard solution (containing each oligosaccharide standard at a concentration of 1. mg/mL).

The filtrates after 3 day reaction and 5 day reaction were sampled out each in an amount of 500 μL, followed by removal of methanol with a centrifugal evaporator and then were dissolved in 100 μL of purified water to prepare methanol soluble matter aqueous solutions. The aqueous solutions were filtered with a 0.45 μm filter and 30 μL of acetonitrile was mixed with 50 μL of each of the filtrates thereby preparing methanol soluble matter analysis samples.

An analysis test was carried out under the following conditions.
Device: LC-10 Avp system, manufactured by Shimadzu Corporation
Column: COSMOSIL Sugar-D 4.6 mm (I.D) x 2, 5 cm, manufactured by Nacalai Tesque
Column temperature: 30°C
Mobile phase: acetonitrile/water=70 vol%/30 vol%
Mobile phase flow rate: 1 mL/min
Detector: RI detector RI2000, manufactured by LSL Lab System
Charge: 10 μL

(Result)

Fig. 4 shows the result of the HPLC analysis of the oligosaccharide standard solution.
Fig. 5 shows the result of the HPLC analysis of the methanol soluble matter after the 3 day reaction.
Fig. 6 shows the result of the HPLC analysis of the methanol soluble matter after the 5 day reaction.

The 3 day reaction methanol soluble matter or the 5 day reaction methanol soluble matter contains no glucose or oligosaccharides and was found to be a mixture including substantially only α-methyl glucoside and β-methyl glucoside.

[Example 2]

(Reaction)

Into a glass container were added 5 g of starch (derived from potato, manufactured by Wako Pure Chemical Industries, Ltd.) heated to 50°C and vacuum-dried with a vacuum pump for 24 hours and 20 ml of distilled methanol, and then the container was placed in a 200 ml stainless-steel pressure tight reactor equipped with a pressure gauge and a resistant reactor equipped with a pressure gauge and a rupture type relief valve (TVS-N2-200 portable reactor, manufactured by Taiatsu Techno) so that the mixture was stirred with a stirrer and allowed to suspend.
After the pressure resistant reactor was sealed, a liquefied $CO_2$ was introduced thereinto, followed by heating with a heater so that the temperature and pressure inside the reactor were 180°C and 8 MPa thereby allowing the carbon dioxide to be in the supercritical state. This state was kept for 21 hours.

(Residue Analysis)

After the predetermined period of time passed, the glass container was taken out and the content therein was filtered to calculate the starch decomposition rate using the following formula.

Starch decomposition rate (%)

=[(weight of charged starch-residue weight)/(weight of charged starch)] x 100

As the result, the decomposition rate of the starch after the 21 hour reaction was 90%.

(Soluble matter analysis : HPLC analysis)

(Result)

The same HPLC analysis of the methanol soluble matter as the above was carried out.
The methanol soluble matter was found to be a mixture including substantially only α-methyl glucoside and β-methyl glucoside.

[Example 3]

(Reaction)

The methanol used in Example 2 was replaced with ethanol, and the same experiment was carried out. The supercritical temperature and pressure of ethanol are 242°C and 6 MPa, respectively.

(Residue analysis)

After the predetermined period of time passed, the glass container was taken out and the content therein was filtered to calculate the starch decomposition rate using the following formula.

$$\text{Starch decomposition rate (\%)} = [(\text{weight of charged starch} - \text{residue weight})/(\text{amount of charged starch})] \times 100$$

As the result, the decomposition rate of the starch after the 21 hour reaction was 88%.

(Soluble matter analysis : HPLC analysis)

(Result)

Fig. 7 shows the result of HPLC analysis of the ethanol soluble matter. The analysis conditions are the same as those described above.
The ethanol soluble matter was found to be a mixture containing mainly α-methyl glucoside and β-methyl glucoside and substantially no ethyl glucosides from saccharide dimer to pentamer.

**Applicability in the Industry**

The present invention can easily produce glucosides having various applications by a simple method and thus has a high industrial utility value.

**Claims**

1. A method for producing glucosides comprising reacting glucose or a polysaccharide comprising glucose as a structural unit with a hydroxyl-containing compound represented by formula (1) below in the presence of a supercritical or subcritical carbon dioxide to produce glucosides represented by formula (2) below:

R-OH (1)

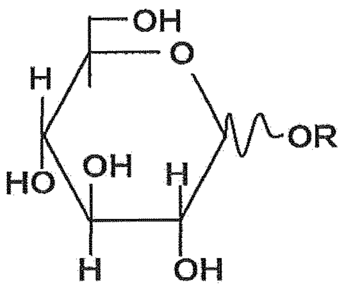

(2)

(in formula (1), R is any substituent, provided that the hydroxyl group in formula (1) bonds to a carbon atom of R and R is excluded from the substituents making the compound of formula (1) sugar, and in formula (2), the wavy line indicates an α-configuration or a β-configuration, and R is as defined with respect to R in formula (1)).

**2.** A method for producing glucosides comprising dissolving or suspending glucose or a polysaccharide comprising glucose as a structural unit in an organic solvent containing a hydroxyl-containing compound represent by formula (1) below and reacting the glucose or polysaccharide with the hydroxyl-containing compound represented by formula (1) in the presence of a supercritical or subcritical carbon dioxide to produce glucosides represented by formula (2) below:

R-OH (1)

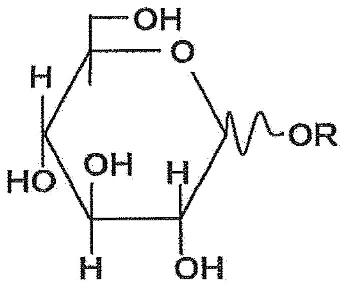

(2)

(in formula (1), R is any substituent, provided that the hydroxyl group in formula (1) bonds to a carbon atom of R and R is excluded from the substituents making the compound of formula (1) sugar, and in formula (2), the wavy line indicates an α-configuration or a β-configuration, and R is as defined with respect to R in formula (1)).

**3.** The method according to claim 1 or 2 wherein the polysaccharide comprising glucose as a structural unit comprises any one selected from amylose, amylopectin and cellulose.

**4.** The method according to any one of claims 1 to 3 wherein the polysaccharide comprising glucose as a structural unit comprises starch.

**5.** The method according to any one of Claims 1 to 4 wherein the hydroxyl-containing compound represented by formula (1) is an alkylalcohol.

**6.** The method according to any one of claims 1 to 5 wherein the hydroxyl-containing compound represented by formula (1) is methanol.

[Fig. 1]

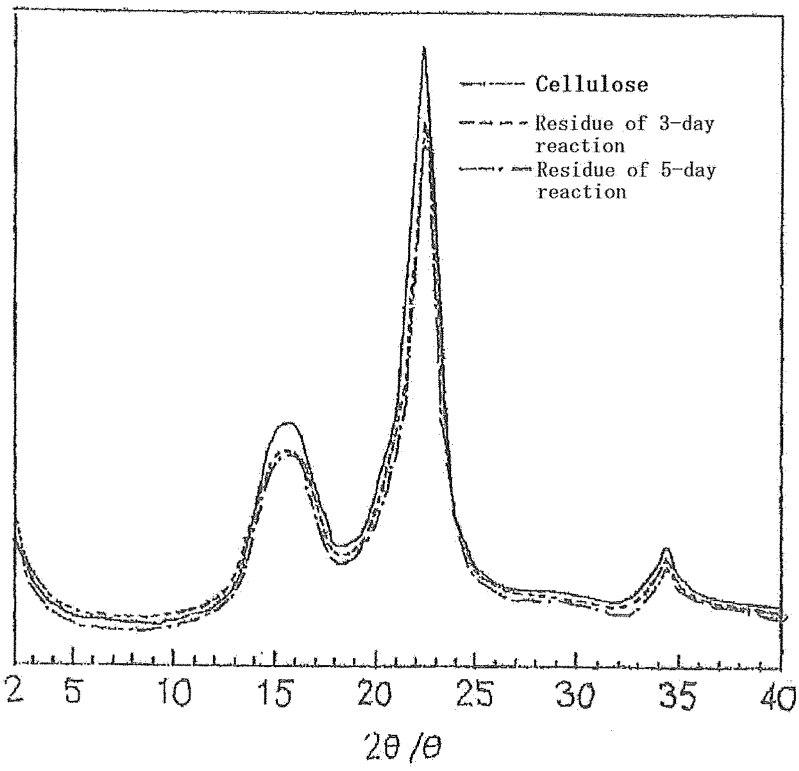
Cellulose
Residue of 3-day reaction
Residue of 5-day reaction
2
5
10
15
20
25
30
35
40
2θ /θ

[Fig. 2]

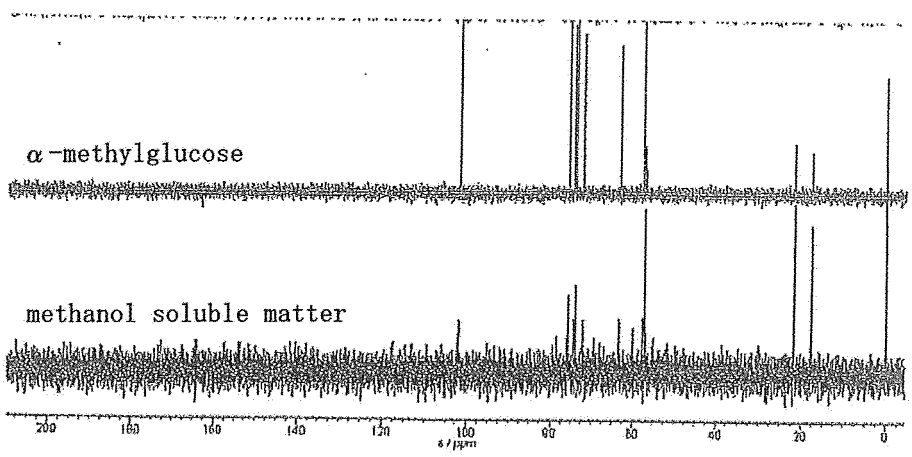
α-methylglucose
methanol soluble matter

[Fig. 3]

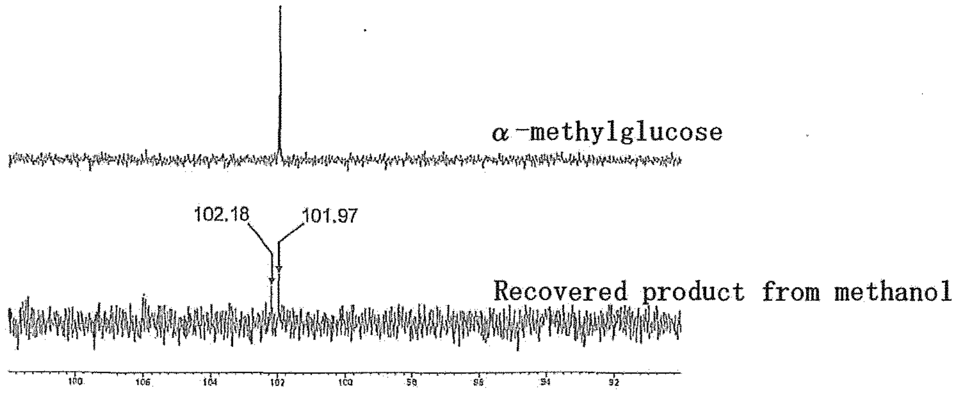
α-methylglucose
102.18
101.97
Recovered product from methanol

[Fig. 4]

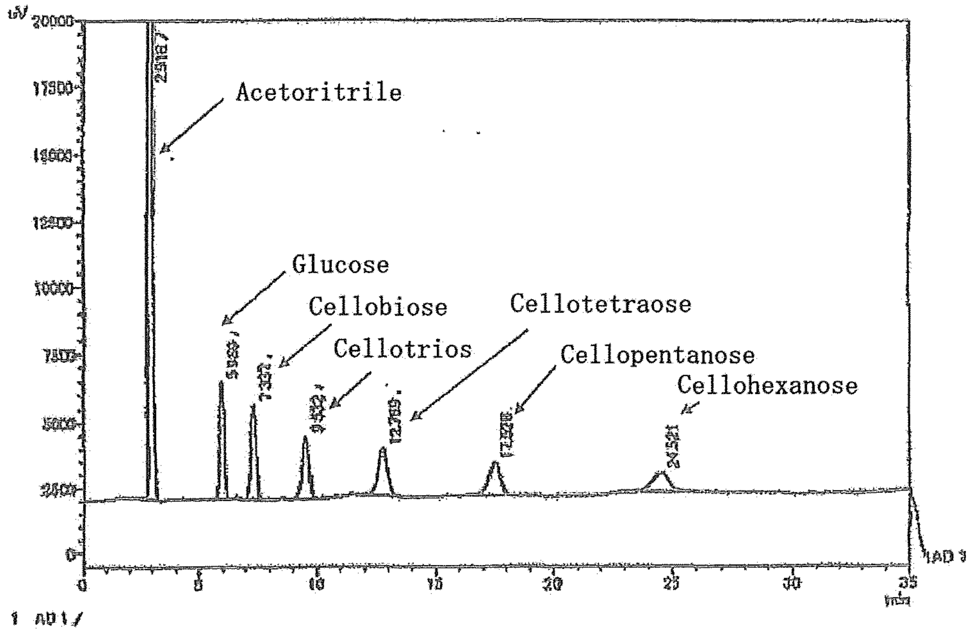
Acetoritrile
Glucose
Cellobiose
Cellotrios
Cellotetraose
Cellopentanose
Cellohexanose

[Fig. 5]

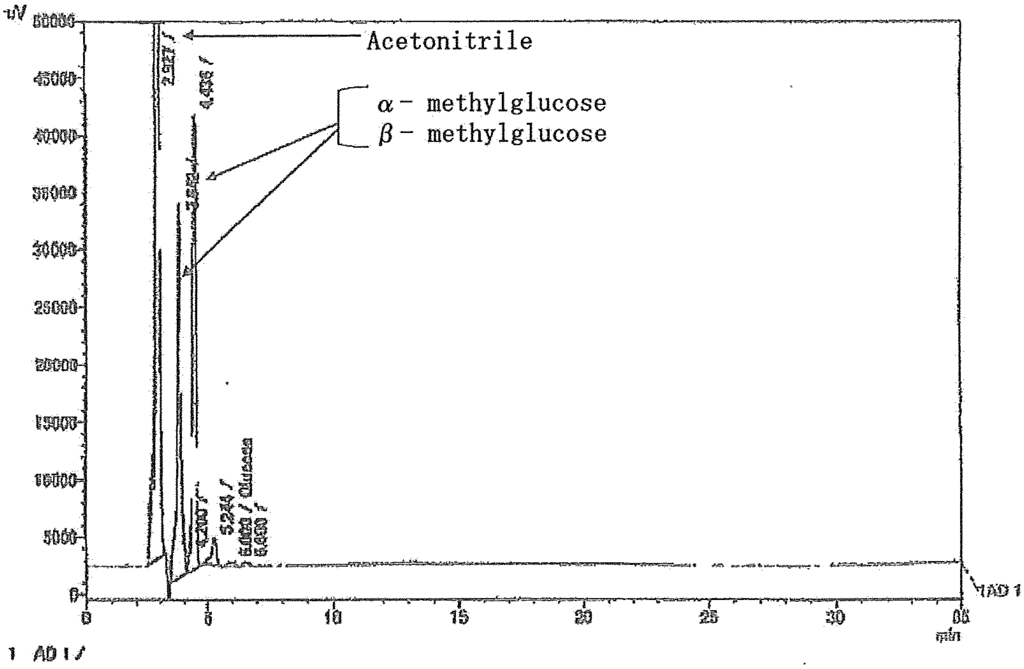
Acetonitrile
α- methylglucose
β- methylglucose

[Fig. 6]

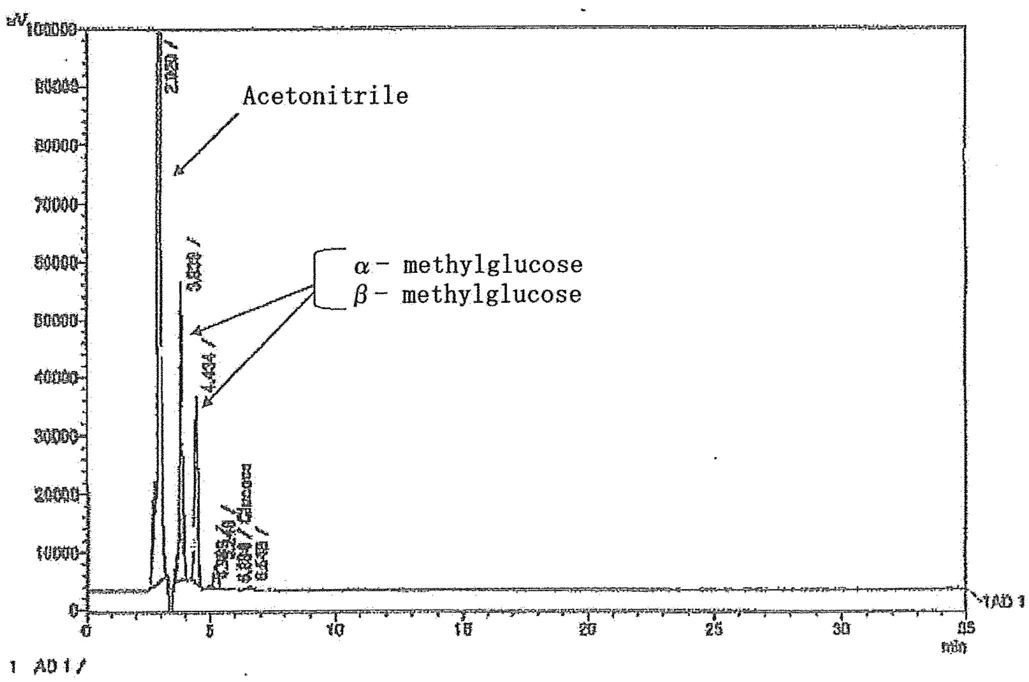
Acetonitrile
α- methylglucose
β- methylglucose

[Fig. 7]

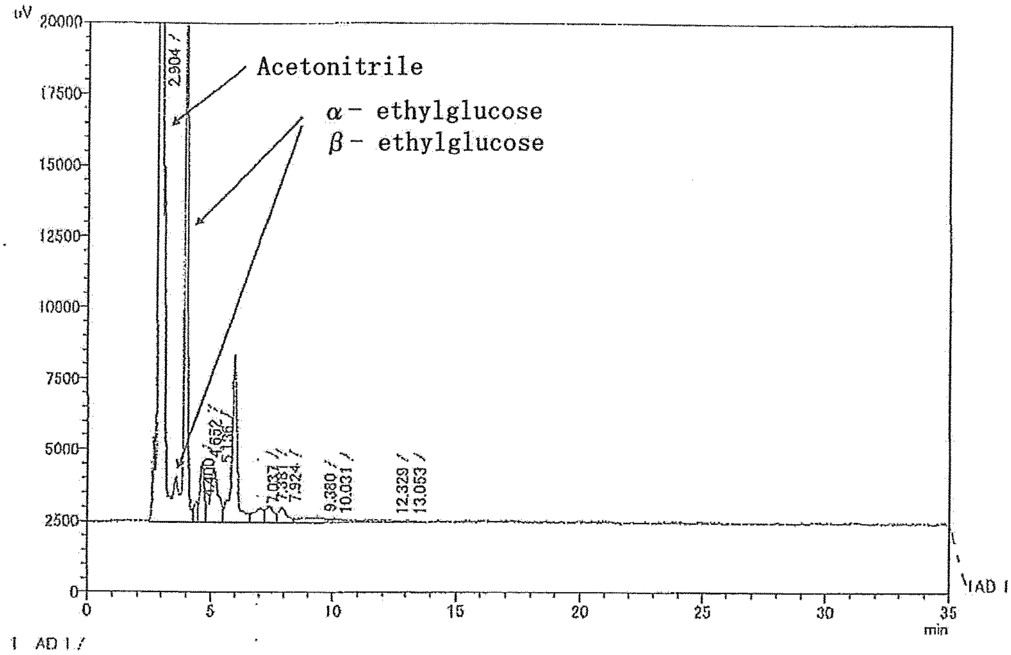

**INTERNATIONAL SEARCH REPORT**

International application No.
PCT/JP2011/061049

A. CLASSIFICATION OF SUBJECT MATTER
*C07H15/04*(2006.01)i, *C07H1/00*(2006.01)i

According to International Patent Classification (IPC) or to both national classification and IPC

B. FIELDS SEARCHED

Minimum documentation searched (classification system followed by classification symbols)
C07H15/04, C07H1/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2011 |
| Kokai Jitsuyo Shinan Koho | 1971-2011 | Toroku Jitsuyo Shinan Koho | 1994-2011 |

Electronic data base consulted during the international search (name of data base and, where practicable, search terms used)
CAplus(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| X | MANOHAR, Balaraman et al., Amyloglucosidase catalyzed syntheses of bakuchiol glycosides in supercritical carbon dioxide, Bulletin of the Korean Chemical Society, 2009, Vol.30, No.8, pp.1760-1766 | 1,2 |
| X | JP 2010-53038 A (Sanyo Chemical Industries, Ltd.), 11 March 2010 (11.03.2010), entire text; particularly, claims; paragraphs [0013] to [0016] (Family: none) | 1,2,5,6 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 21 July, 2011 (21.07.11) | 02 August, 2011 (02.08.11) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No.
PCT/JP2011/061049

C (Continuation). DOCUMENTS CONSIDERED TO BE RELEVANT

| Category* | Citation of document, with indication, where appropriate, of the relevant passages | Relevant to claim No. |
|---|---|---|
| A | JP 2005-336163 A (Goldschmidt GmbH), 08 December 2005 (08.12.2005), entire text & EP 1627882 A1 & US 2005/0261484 A1 & DE 102004025195 A & CN 1699387 A | 1-6 |
| A | JP 60-1196 A (Daicel Chemical Industries, Ltd.), 07 January 1985 (07.01.1985), entire text & US 4683297 A | 1-6 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2275892 A
- JP 9031089 A
- JP 2002017395 A
- JP 2002017396 A
- JP 5031000 A
- JP 2006263527 A
- JP 2005296906 A


### Non-patent literature cited in the description


- **Y. ISHIKAWA ; S. SAKA.** *Cellulose,* 2001, vol. 8, 189
- **HIROSHI ICHIYANAGI ; MUTSUHISA FURUKAWA ; KEN KOJIO ; SUGURU MOTOKUCHO.** *Polymer Preprints, Japan,* 2009, vol. 58 (2), 5387
- **SUGURU MOTOKUCHO ; SHINGO MUKAI ; KEN KOJIO ; MUTSUHISA FURUKAWA.** *Polymer Preprints, Japan,* 2007, vol. 56 (1), 2359